# EUROPÄISCHE PATENTSCHRIFT

(11) **EP 3 402 576 B1**
(45) Veröffentlichungstag und Bekanntmachung des Hinweises auf die Patenterteilung: **28.07.2021**
(21) Anmeldenummer: 16812678.7
(22) Anmeldetag: 16.12.2016
(51) Int. Cl.: A61Q 19/04, A61Q 19/08, A61Q 17/04, A61Q 19/00, A61K 8/60

(54) **NOREUGENIN-GLYKOSID-DERIVATE**
NOREUGENIN GLYCOSIDE DERIVATIVES
DÉRIVÉ DE NOREUGÉNINE GLYCOSIDE

(30) Priorität: 15.01.2016 EP 16151433
(43) Veröffentlichungstag der Anmeldung: 21.11.2018
(73) Patentinhaber: Universität Hamburg, 20148 Hamburg (DE)
(72) Erfinder: CAROLA, Christophe, 64625 Bensheim (DE); SCHULTE, Michael, 65474 Bischofsheim (DE); RABAUSCH, Ulrich, 20249 Hamburg (DE); ROSENFELD, Henning, 22395 Hamburg (DE); STREIT, Wolfgang, 24248 Moenkeberg (DE)
(74) Vertreter: Stüven, Ralf
(86) Internationale Anmeldenummer: PCT/EP2016/002119
(87) Internationale Veröffentlichungsnummer: WO 2017/121445

(56) Entgegenhaltungen:
- WO-A1-2007/087956
- WO-A1-2014/191524
- DE-A1- 10 337 863
- KALIA RAJWANT K ET AL: "Understanding Tecomella undulata: an endangered pharmaceutically important timber species of hot arid regions", GENETIC RESOURCES AND CROP EVOLUTION, KLUWER, DORDRECHT, NL, Bd. 61, Nr. 7, 19. Juni 2014 (2014-06-19), Seiten 1397-1421, XP035402706, ISSN: 0925-9864, DOI: 10.1007/S10722-014-0140-3 [gefunden am 2014-06-19]
- DATABASE TKDL [Online] CSIR India; 1. Januar 1928 (1928-01-01), NN: "Bh'tabhairavarasa a(1)", XP002766623, Database accession no. RD/707
- DATABASE TKDL [Online] CSIR India; 1. Januar 1999 (1999-01-01), NN: "Nyagrodhçdyamgh"tam", XP002766624, Database accession no. RG/683
- LUBICA LACIKOVA ET AL: "Antiproliferative, Cytotoxic, Antioxidant Activity and Polyphenols Contents in Leaves of Four Staphylea L. Species", MOLECULES ONLINE, Bd. 14, Nr. 9, 28. August 2009 (2009-08-28), Seiten 3259-3267, XP055338940, DE ISSN: 1433-1373, DOI: 10.3390/molecules14093259
- Y.-B. WANG ET AL: "Chromone glycosides from Knoxia corymbosa", JOURNAL OF ASIAN NATURAL PRODUCTS RESEARCH, Bd. 8, Nr. 7, 1. Oktober 2006 (2006-10-01) , Seiten 663-670, XP055338977, CH ISSN: 1028-6020, DOI: 10.1080/10286020500246303
- LIFENG HAN ET AL: "Triglyceride accumulation inhibitory effects of new chromone glycosides from Drynaria fortunei", NATURAL PRODUCT RESEARCH, Bd. 29, Nr. 18, 17. September 2015 (2015-09-17), Seiten 1703-1710, XP055339792, GB ISSN: 1478-6419, DOI: 10.1080/14786419.2014.998216
- SOON-JOO SOHN ET AL: "Chemical Constituents of the Leaves of Staphylea bumalda | Korea Science", NATURAL PRODUCT SCIENCES, Bd. 10, Nr. 4, 1. Januar 2004 (2004-01-01) , Seiten 173-176, XP055338850, Korea
- V.K. GUJRAL ET AL: "A new chromone glucoside from Tecomella undulata", PHYTOCHEMISTRY, Bd. 18, Nr. 1, 1. Januar 1979 (1979-01-01) , Seiten 181-182, XP055338836, GB ISSN: 0031-9422, DOI: 10.1016/S0031-9422(00)90945-2
- SURESH KUMAR ET AL: "In vivo anti-hyperglycemic and antioxidant potentials of ethanolic extract from Tecomella undulata", DIABETOLOGY & METABOLIC SYNDROME, BIOMED CENTRAL LTD, LONDON, UK, Bd. 4, Nr. 1, 6. Juli 2012 (2012-07-06), Seite 33, XP021108263, ISSN: 1758-5996, DOI: 10.1186/1758-5996-4-33
- Junichi Kitajima ET AL: "Pharmaceutical Society of Japan NII-Electronic Library Service Moneterpenoid Glucosidesof Cnidium monnieri Fruit", Chem. Pharm. Bull, 1. Januar 1999 (1999-01-01), Seiten 639-642, XP055338865, Gefunden im Internet: URL:http://ci.nii.ac.jp/lognavi?name=nels& lang=en&type=pdf&id=ART0004118135 [gefunden am 2017-01-25]
- DATABASE MEDLINE [Online] US NATIONAL LIBRARY OF MEDICINE (NLM), BETHESDA, MD, US; September 2007 (2007-09), XUE JUN-YI ET AL: "[Studies on the chemical constituents of Adina pilulifera].", XP002766625, Database accession no. NLM18236749 & ZHONG YAO CAI = ZHONGYAOCAI = JOURNAL OF CHINESE MEDICINAL MATERIALS SEP 2007, Bd. 30, Nr. 9, September 2007 (2007-09), Seiten 1084-1086, ISSN: 1001-4454

## Beschreibung

Die vorliegende Erfindung betrifft die Verwendung von Noreugenin-Glykosid-Derivaten der Formel I und II als Selbstbräunungssubstanz oder zur Steigerung der Melaninsynthese, Verbesserung des Melanintransports und/oder Verbesserung der Verteilung von Melanin in suprabasalen Schichten, Zubereitungen enthaltend Noreuginin-Glykosid-Derivaten der Formel I, II und III, sowie Noreuginin-Glykosid-Derivaten der Formel I, II und 111 selbst.

Der Trend weg von der vornehmen Blässe hin zur "gesunden, sportlich braunen Haut" ist seit Jahren ungebrochen. Um einen gebräunten Teint zu erzielen, setzen die Menschen ihre Haut der Sonnenstrahlung aus, da diese eine Pigmentierung durch eine Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko, an Hautkrebs zu erkranken. Die übermäßige Einwirkung der UVB- und UVA-Strahlung (Wellenlänge: 320-400 nm) erzeugt hochreaktive Radikalspezies, die sich auch nach Beendigung der Bestrahlung weiter vermehren und als deren Folge es zu Faltenbildung und Hautalterung kommt.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird. Melanin schützt die Zellkerne vor weiterer Bestrahlung und den davon verursachten negativen Auswirkungen auf die Zell-DNA.

Je nach chemischer Zusammensetzung der biochemisch gebildeten Pigmente wird zwischen dem bräunlich-schwarzen Eumelanin und dem rötlich-gelben Phäomelanin unterschieden. Der beobachtete Hautfarbton wird vom Verhältnis dieser beiden Melaninarten bestimmt.

Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt.

Diese Verbindungen weisen als chemisches Strukturmerkmal Keto- bzw. Aldehydgruppen in Nachbarschaft zu Alkoholfunktionen auf und gehören überwiegend zur Substanzklasse der Zucker. Besonders häufig eingesetzte Selbstbräunungssubstanzen sind 1,3-Dihydroxyaceton (DHA), das in einer Menge von 700t/a verwendet wird, und Erythrulose.

Selbstbräuner können mit den Proteinen und Aminosäuren der Hornschicht der Haut im Sinne einer Maillard-Reaktion oder über eine Michael Addition umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Diese Farbprodukte besitzen allerdings selbst keine UV-absorbierenden Eigenschaften, sodass bei Sonnenexposition ein zusätzlicher Sonnenschutz (Bekleidung, Hut, UV-Filter) erforderlich wird.

Im Gegensatz zu "sonnengebräunter" Haut ist die so gebräunte Haut nicht gegen Sonnenbrand geschützt.

Es besteht daher weiterhin ein Bedarf nach dermatologisch verträglichen hautfärbenden Substanzen, die sich zum Einsatz in kosmetischen und/oder dermatologischen Zubereitungen oder Medizinprodukten eignen und die die natürliche Bräunung der Haut durch Steigerung der Melaninsynthese verstärken und gleichzeitig einen besseren Hauteigenschutz bzw. Sonnenschutz, insbesondere gegen UVB-Strahlung, ermöglichen.

Bislang sind nur wenige solcher Substanzen bekannt. WO 2012/097857 A1 beschreibt beispielsweise die Verwendung von 7-Acyloxy-Chromen-4-on-Derivaten als Selbstbräunungssubstanzen, die die natürliche Hautbräunung anregen. Darüber hinaus beschreibt auch die WO 2007/087956 A1 Chromen-4-on-Derivate als Selbstbräungungssubstanz.

In der DE 103 37 863 A1 sind Chromen-4-on-Derivate zur Pflege, Konservierung oder Verbesserung des allgemeinen Zustandes der Haut oder Haare beschrieben, die auch zur Prophylaxe gegen Pigmentstörungen und zur Reduktion oder Verhinderung schädigender Effekte von UV-Strahlen auf die Haut geeignet sein sollen.

In einem Review von Kalia et al. 2014 (Kalia, R.K., Rai, M.K., Sharma, R., Bhatt, R.K., 2014, Understanding Tecomella undulata: an endangered pharmaceutically important timber species of hot arid regions", Genet. Resour. Corp. Evol. 61, 1397-1421) zu *Tecomella undulata* sind zahlreiche medizinische Anwendungen der Pflanze beschrieben, u.a. die Verwendung der Rinde bei Leucoderma. Kalia et al. 2014 erwähnen das Vorhandensein von Undulatosid A (2-Methyl-5,7-dihydroxychromon-7-O-β-D-glucopyranosid) in der Rinde von *Tecomella undulata.*

Unter den Datenbankeinträgen RD/707 und RG/683 der "Traditional Knowledge Digital Library" (TKDL), Indien, Council of Scientific & Industrial Research (CSIR), sind Zusammensetzungen von *Tecomella undulata* mit der Bezeichnung "Bh'tabhairavarasa a(1)" und "Nyagrodhçdyamgh"tam"" zur oralen Verabreichung beschrieben, die traditionell u.a. zur Behandlung von Lepraund Dermatosen sowie zur Behandlung von Schmerzen im Bauchraum verwendet werden.

WO2007/087956 offenbart Chromen-4-on-Derivate als Selbstbräunungssubstanzen.

Die der vorliegenden Erfindung zugrunde liegende Aufgabe bestand daher in der Bereitstellung von weiteren Selbstbräunungssubstanzen, die die biologische Hautbräunung anregen.

Überraschenderweise wurde festgestellt, dass sich Noreugenin-Glykosid-Derivate als Selbstbräunungssubstanzen eignen.

Im Sinne der Erfindung wird der Begriff Selbstbräunungswirkstoff synonym zu Selbstbräunungssubstanz oder Selbstbräunersubstanz verwendet. 7-O-β-D-glucosyl-noreugenin (7-(β-D-glucopyranosyloxy)-5-hydroxy-2-methyl-4*H*-1-Benzopyran-4-on oder Undulatoside A) **(Ia)** kommt natürlich in verschiedenen Pflanzen vor, wie beispielsweise *Staphylea bumalda* (J.S. Soon et al., Natural Product Science (2004), 10(4) 173-176), *Tecomella undulata* (V. K. Gujral et al., Indian Journal of Chemistry Section B-Organic Chemistry Including Medicinal Chemistry (1979), 17, 40-41; S. Kumar et al., (2012), Diabetology & Metabolic Syndrome 4, 33), *Adina pilulifera* (H. W.-h. GUO Yue-wei et al. (2003), Chinese Journal of Magnetic Resonance 20, 265-269) oder in den Früchten von *Cnidium monnieri* (J. Kitajina et al. Chemical & Pharmaceutical Bulletin (1999), 47(5) 639-642). Eine kosmetische Anwendung dieser Substanz ist nicht bekannt.

Ein erster Gegenstand der vorliegenden Erfindung ist daher die Verwendung mindestens einer Verbindung der Formel I oder II 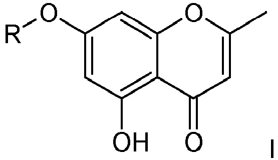 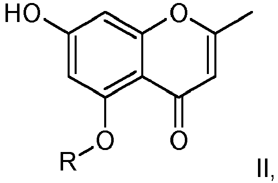 wobei die Reste R für Rhamnosyl stehen, wobei die Reste jeweils als D- oder L-Enantiomer und als α- oder β-Anomer vorliegen können,
als Selbstbräunungssubstanz.

Grundsätzlich sind im Sinne der vorliegenden Erfindung von der Bezeichnung "Verbindung nach Formel I oder II" auch die Salze der jeweiligen Verbindungen nach Formel I oder II umfasst. Zu den bevorzugten Salzen gehören dabei insbesondere Alkali- und Erdalkalimetallsalze, Zink- sowie Ammonium-Salze, insbesondere jedoch Natrium- und Kalium-Salze.

Die Verbindungen der Formel I oder II können in Selbstbräunungsprodukten als Selbstbräunungssubstanz und/oder zur Steigerung der Melaninsynthese in der Haut, zur Verbesserung des Melanintransports und/oder zur Verbesserung der Verteilung von Melanin in suprabasalen Schichten und/oder zum Schutz der Haut vor schädigenden UV-Strahlen eingesetzt werden.

Ebenfalls Gegenstand der vorliegenden Erfindung ist die Verwendung einer Verbindung der Formel I oder II, wie zuvor beschrieben, zur Steigerung der Melaninsynthese, Verbesserung des Melanintransports und/oder Verbesserung der Verteilung von Melanin in suprabasalen Schichten.

Verbindungen der Formel I oder II steigern die Melaninsynthese und verbessern den Melanintransport von den Melanocyten zu den Keratinocyten. Dies wirkt sich auf die Farbe der Haut aus und bewirkt einen Bräunungseffekt.

Die Zuckerreste sind in den Verbindungen der Formel I oder II jeweils über die OH-Gruppe am anomeren C-Atom an den Noreugenin-Rest gebunden.

Weiterhin bevorzugt ist die Verbindung der Formel I 7-O-α-L-rhamnosylnoreugenin (7-[(6-deoxy-α-L-mannopyranosyl)oxy]-5-hydroxy-2-methyl-4*H-*1-Benzopyran-4-on) **(Ib)** ist und die Verbindung der Formel II 5-O-α-L-rhamnosyl-noreugenin (5-[(6-deoxy-α-L-mannopyranosyl)oxy]-7-hydroxy-2-methyl-4*H*-1-Benzopyran-4-on) **(IIa)** ist:

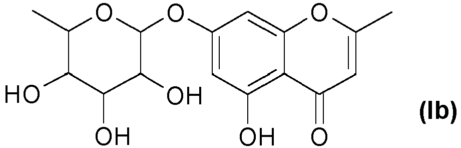

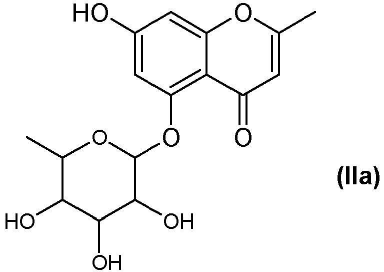

Die Verbindungen der Formel I oder II können durch enzymatische Glykosylierung von Noreugenin hergestellt werden. Geeignete Enzyme sind beispielsweise in WO 2014/191524 A1 beschrieben:
Bei den Enzymen handelt es sich um die Glykosyltransferasen MgtB und GtfC aus WO 2014/191524 A1. Für die Übertragung von Glucoseresten wird MgtB verwendet, für Rhamnosetransfer das Enzym GtfC, welches jeweils rekombinant in *Escherichia coli* exprimiert werden kann. Dabei kann die enzymatische Reaktion als ganzzellkatalytischer Prozess oder mit isolierten Enzymen und Reinsubstanzen erfolgen. Die Ganzzellkatalyse hat den Vorteil, dass notwendige aktivierte Zucker, UDP-Glucose oder TDP-Rhamnose, vom Stoffwechsel der Mikroorganismen permanent nachgebildet werden. Hierbei bereitet es dem Fachmann keine Schwierigkeiten, die geeigneten Reaktionsbedinungen auszuwählen.

Die Verbindungen der Formel I oder II können neben der Bräunungswirkung auch eine antioxidante Wirkung haben und weisen eine gute Hautverträglichkeit auf. Zusätzlich sind bevorzugte der hier beschriebenen Verbindungen farblos oder nur schwach gefärbt und führen so nicht oder nur in geringer Weise zu Verfärbungen der Zubereitungen. Die bevorzugten Verbindungen weisen außerdem eine verbesserte Löslichkeit in der wässrige Phase einer Zubereitung auf.

Damit die Verbindungen der Formel I oder II ihre positive Wirkung auf die Haut besonders gut entwickeln können, kann es bevorzugt sein, die Verbindungen der Formel I oder II, wie zuvor beschrieben, in tiefere Hautschichten eindringen zu lassen. Dazu stehen mehrere Möglichkeiten zur Verfügung. Zum einen können die Verbindungen der Formel I oder II eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I oder II durch die äußeren Hautschichten ermöglichen. Schließlich ist auch ein systemischer Transport der Verbindungen der Formel I oder II denkbar. Die Zubereitung wird dann beispielsweise so gestaltet, dass sie für eine orale Gabe geeignet ist.

Die erfindungsgemäße Verwendung erfolgt bevorzugt nicht-therapeutisch.

Ein weiterer Gegenstand der vorliegenden Erfindung ist eine Zubereitung enthaltend mindestens eine Verbindung der Formel I oder II oder deren bevorzugte Ausführungsformen, wie zuvor beschrieben definiert, sowie einen kosmetisch geeigneten Träger.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Im Sinne der vorliegenden Erfindung wird neben dem Begriff Zubereitung gleichbedeutend auch der Begriff Mittel oder Formulierung verwendet.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Bevorzugt handelt es sich um eine kosmetische oder pharmazeutische Zubereitung; besonders bevorzugt handelt es sich um eine kosmetische Zubereitung.

Die mindestens eine Verbindung der Formel I oder II wird in den erfindungsgemäßen Zubereitungen typischerweise in Mengen von 0,01 bis 10 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung entsprechend auszuwählen.

Ferner können die erfindungsgemäßen Zubereitungen mindestens eine weitere Selbstbräunungssubstanz als weiteren Inhaltsstoff enthalten. Dabei kann es sich sowohl um einen Selbstbräuner handeln, der mit den Aminosäuren der Haut im Sinne einer Maillard-Reaktion oder über eine Michael-Addition reagiert, als auch um einen sogenannten Melanogenese-Promotor oder Propigmentierungswirkstoff, der die natürliche Bräunung der Haut fördert.

Als vorteilhafte Selbstbräunungssubstanzen können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Propigmentierungsstoffe können prinzipiell alle dem Fachmann bekannten Wirkstoffe sein. Beispiele hierfür sind Glycyrrhetinsäure, Melanocytenstimulierendes Hormon (alpha-MSH), Peptidanaloga, Thymidin-Dinucleotide, L-Tyrosin und dessen Ester, bicyclische Monoterpendiole (beschrieben in Brown et al., Photochemistry and Photobiology B: Biology 63 (2001) 148-161) oder 7-Acyloxy-Chromen-4-on-Derviate (beschrieben in WO 2012/097857 A1), insbesondere Hexadecansäure 5-hydroxy-2-methyl-4-oxo-4H-chromen-7-yl ester (vertrieben durch die Firma Merck KGaA, Darmstadt, Deutschland unter dem Namen Ronacare® Bronzyl™).

Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung auch Formaldehydfänger sowie gegebenenfalls Flavonoide zur Verbesserung des Geruches enthalten.

Die erfindungsgemäße Zubereitung, welche eine Selbstbräunungssubstanz und eine Verbindung der Formel I oder II kombiniert, hat gegenüber einem Selbstbräunungsprodukt ohne Zugabe einer Verbindung der Formel I oder II folgende Vorteile:
- Beschleunigung der Bräunungs-Reaktion,
- Verlängerung der Bräunungs-Reaktion aufgrund der indirekten Bräunungs-Reaktion (UV-freie Bräunungsverlängerung),
- Verstärkung der Bräunungs-Reaktion,
- Vermeidung von ungleichmäßiger Bräunung durch ungeschicktes Auftragen,
- die erzielte Bräunung kommt der natürlichen Bräunung nahe,
- Verbesserung des Schutzes vor UV-Strahlung.

Die erfindungsgemäßen Zubereitungen können auch neben den Verbindungen der Formel I oder II zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.
Salicylate: Homosalate vetrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.
β,β-Diphenylacrylate Derivate: Octocrylene, z. B. vertrieben unter dem Namen "Eusolex® OCR" von der Firma Merck", "Uvinul N539" von der Fa. BASF, Etocrylene, z. B. vertrieben unter dem Namen "Uvinul N35" von der BASF.
Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40" ; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.
Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.
Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.
Phenylbenzotriazol Derivate: Drometrizole trisiloxane, z. B. vertrieben unter dem Namen "Silatrizole" von der Fa. Rhodia Chimie, Methylenebis(benzotriazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. BASF.
Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V, oder Trisbiphenyltriazin, vertrieben unter dem Namen Tinosorb® A2B von der Firma BASF.
Anthranilin Derivate: Menthyl anthranilate, z. B. vertrieben unter dem Namen "Neo Heliopan MA" von der Fa. Symrise.
Imidazol Derivate: Ethylhexyldimethoxybenzylidenedioxoimidazoline propionat.
Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.
4,4-Diarylbutadien Derivate: 1,1-Dicarboxy(2,2'-dimethylpropyl)-4,4-diphenylbutadiene.
Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

Piperazinderivate wie beispielsweise die Verbindung 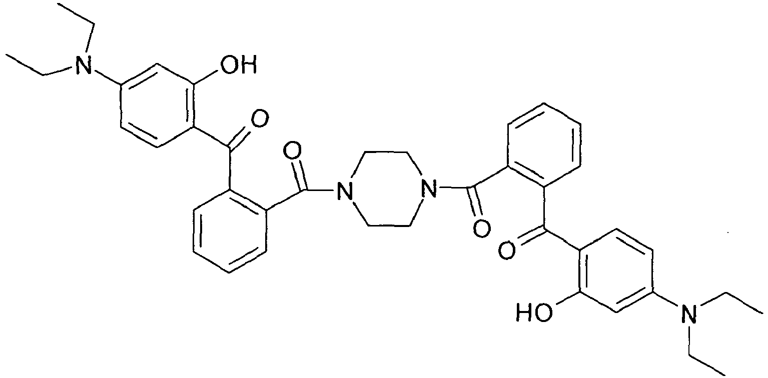 oder die UV-Filter der folgenden Strukturen 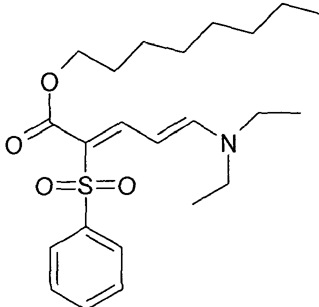 oder

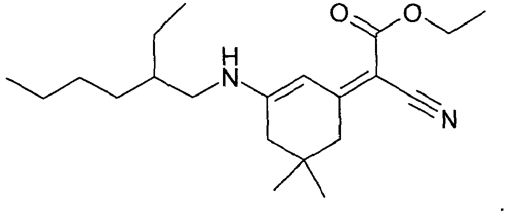

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

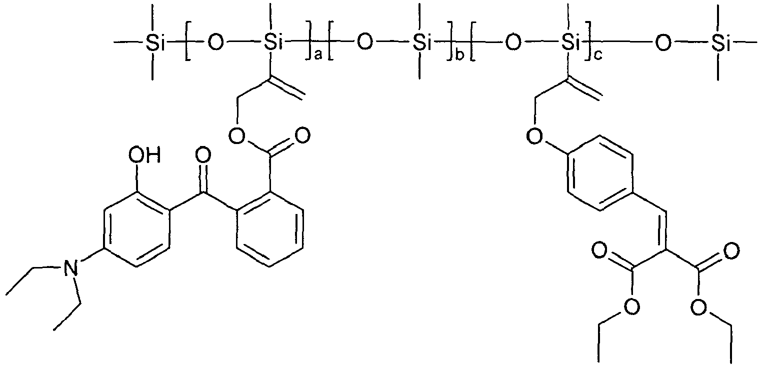

Die in der Liste aufgeführten Verbindungen sind nur als Beispiele aufzufassen. Selbstverständlich können auch andere UV-Filter verwendet werden.

Geeignete organische UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I oder II sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandioxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B . das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die erfindungsgemäß mindestens eine Verbindung der Formel I oder II enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-ageing-, Anti-Falten-, Anti-Schuppen-, Anti-Akne-, Anti-Cellulite-Wirkstoffen, Deodorants oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercetin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 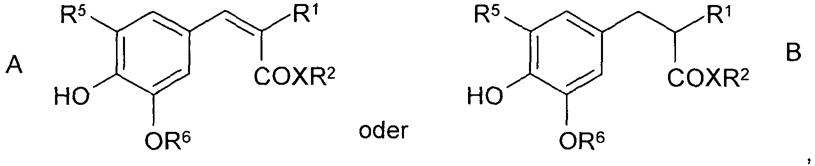 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Gegenstand der vorliegenden Erfindung ist auch ein Verfahren zur Herstellung einer Zubereitung, wie zuvor beschrieben, dadurch gekennzeichnet, dass mindestens eine Verbindung der Formel I, II oder III mit einem für topische Zubereitungen geeigneten Träger und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-ÖI (W/O) oder vom Typ Öl-in-Wasser (O/W), eine multiple Emulsion, beispielsweise vom Typ Waser-in-ÖI-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, XTend 226 (L'Oréal), Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Ein weiterer Gegenstand der vorliegenden Erfindung sind Verbindungen der Formel I oder II 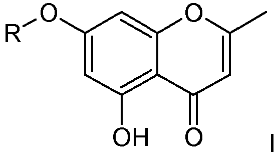 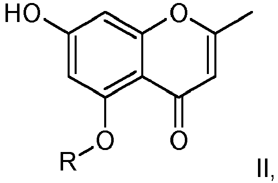 wobei R für Rhamnosyl steht, wobei die Reste jeweils als D- oder L-Enantiomer und als α- oder β-Anomer vorliegen können.

In einer ganz besonders bevorzugten Ausführungsform ist die Verbindung der Formel I oder II ausgewählt aus den Verbindungen 7-O-α-L-rhamnosylnoreugenin (Ib) oder 5-O-α-L-rhamnosyl -noreugenin (Ila).

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen.

Weitere wichtige Merkmale und Vorteile der Erfindung ergeben sich aus den Unteransprüchen und aus den Beispielen.

### Abbildungen:

Fig 1: Biotransformation nach 6 Stunden zur Herstellung von Noreugenin-Rhamnosiden. Die annotierten Peaks von links nach rechts sind. Noreugenin 5-Rhamnosid, Noreugenin 7-Rhamnosid, Noreugenin bei den entsprechenden Retentionszeiten 46 min, 55 min und 60 min.
Fig. 2: RP18 Chromatogramm von Noreugenin 5-Rhamnosid.
Fig. 3: RP18 Chromatogramm von Noreugenin 7-Rhamnosid.
Fig. 4: Ion Scan von Noreugenin Rhamnosiden.

Die Beispiele sollen die vorliegende Erfindung näher erläutern, ohne deren Umfang zu beschränken.

### Beispiele:

### Beispiel 1: Versuchsdurchführung: Beurteilung der Bräunungseigenschaften der Prüfpräparate unter Verwendung von rekonstituierter menschlicher Epidermis mit Melanozyten des Phototyps II

Die Epidermis mit Melanozyten des Phototyps II wird von Tag 0 bis Tag 4 in einem Air/Liquid-Interface-System (unmittelbar an der Luft-Flüssigkeitsgrenzschicht) in Kulturmedium zur vollständigem Differenzierung rekonstituiert. Anschließend wird die Kultur von Tag 4 bis Tag 14 im selben Medium, aber ohne Rinderhypophysenextrakt (Bovine Pituitary Extract, BPE) fortgeführt. Die Prüfpräparate werden von Tag 4 bis Tag 14 in das Kulturmedium appliziert. Das Medium und die Prüfpräparate werden am Tag 4, Tag 7 und Tag 11 jeweils frisch zugegeben. DMSO und THF werden parallel getestet und IBMX (100 µM) dient als positive Referenz.

### Beispiel 2: Versuchsdurchführung: Semiquantifizierung des Melaningehalts durch Fontana-Masson-Färbung und Bildanalyse

Nach Paraffineinbettung wird eine Fontana-Masson-Färbung der Gewebeschnitte vorgenommen. Die Objektträger werden mit einem Spezialmedium bedeckt und mit einem Lichtmikroskop vom Typ Leica DM2000 mit Digitalkamera untersucht. Nach der Färbung werden Bilder aufgenommen und eine Quantifizierung durch Bildanalyse (auf der Grundlage von 12 Bildern) durchgeführt. Zur Quantifizierung des Melaningehalts in jedem Bild wird die Leica QWin3-Software verwendet. Es werden 2 Messungen durchgeführt. Die erste Messung gibt die Gesamtintensität der Färbung wieder, d. h. es wird ein helleres oder dunkleres Melanin reflektiert. Die zweite Messung bezieht sich auf die markierte Bildfläche, d. h. den melaninhaltigen Bereich der Epidermis. Bei diesen Messungen werden alle Zellschichten mit Ausnahme des *Stratum corneum* einbezogen.

### Beispiel 3: Bräunungseigenschaften der Substanzen Ia, Ib und IIa Die Durchführung erfolgt wie unter Beispeil 1 und 2 beschrieben.

Nach Bestimmung der zytotoxischen Konzentrationen wird Substanz (I**a**, nicht gemäß der Erfindung) bei 8 µM, Substanz (**Ib**) bei 40 µM (Lösungsmittel: 0,1 % THF) und Substanz **(IIa)** bei 20 µM (Lösungsmittel: 0,1 % DMSO) getestet.

| **Präparate** | **Quantifizierung nach Fontana-Masson-Färbung** |
|---|---|
| *Quantifizierung relativ zur unbehandelten Kontrolle* | |
| IBMX (100 µM) | **152** % (p < 0,05) |
| Substanz (I**a**, 8 µM) | **125%** |
| Substanz (I**b**, 40 µM) | **174** % (p < 0,05) |
| Substanz (II**a**, 20 µM) | **143** % (p < 0,05) |

Für IBMX wird eine signifikante Erhöhung des Melaningehalts relativ zur unbehandelten Kontrolle festgestellt. Dasselbe gilt für Substanz (**Ib**) relativ zu THF und Substanz **(IIa)** relativ zu DMSO. Für Substanz (I**a**, nicht gemäß der vorliegenden Erfindung) wurde eine eindeutige Tendenz zur verstärkten Pigmentierung festgestellt.

### Beispiel 4: Bräunungseigenschaften der Substanzen Ib, IIa, eines Gemischs aus beiden Substanzen und Rhamnose allein

In einem zweiten Versuch werden die Substanzen (**Ib**) und (II**a**), ein Gemisch aus beiden Substanzen und zum Vergleich der Zuckerrest allein (Rhamnose) getestet.

Die Durchführung erfolgt wie unter Beispiel 1 und 2 beschrieben.

Nach Bestimmung der zytotoxischen Konzentrationen dieser Substanzen wird Substanz (**Ib**) bei 40 µM in DMSO (0,1 %), Substanz **(IIa)** bei 20 µM in DMSO (0,1 %), das Gemisch aus (I**b**)/(II**a**) bei 40/20 µM in DMSO (0,1 %) und Rhamnose bei 40 µM in THF (0,2 %) getestet.

Die erhaltenen Ergebnisse bestätigten die Ergebnisse der ersten Studie.

| **Präparate** | **Quantifizierung nach Fontana-Masson-Färbung** |
|---|---|
| *Quantifizierung relativ zur unbehandelten Kontrolle* | |
| IBMX (100 µM) | **150** % (p < 0,01) |
| Rhamnose (40 µM) | **109 %** |
| Substanz (I**b**, 40 µM) | **167** % (p < 0,01) |
| Substanz (II**a**, 20 µM) | **147** % (p < 0,05) |
| Gemisch (I**b**/II**a**, 40 µM/20 µM) | **140** % |

Für IBMX wirde eine signifikante Erhöhung des Melaningehalts relativ zur unbehandelten Kontrolle festgestellt. Dasselbe gilt für Substanz (**Ib**) relativ zu DMSO und Substanz (II**a**) relativ zu DMSO. Für das Gemisch (I**b**/II**a**) wirde eine eindeutige Tendenz zur verstärkten Pigmentierung festgestellt. Bei Rhamnose ist die Tendenz zur verstärkten Melaninsynthese schwach ausgeprägt.

### Beispiel 5: Herstellung von Noreugenin 5-Rhamnosid (IIa) und Noreugenin 7-Rhamnosid (Ib)

Die Biotransformation wird in einem fermentativen Prozess in 3L Erlenmeyer Schüttelkolben mit einem Fermentationsvolumen von 500 mL vollzogen. Dazu werden die transgenen Stämme *E. coli* BL21 (DE3) pET19::*mgtB* für die Glucosylierung oder *E. coli* Rosetta gami 2 (DE3) pET19::*gtfC* in Luria-Bertani (LB)-Komplexmedium bei 28°C zu einer optischen Dichte (OD₆₀₀) von 0,8 herangezogen. Durch Zugabe von 50 µM Isopropyl-β-D-thiogalactopyranoside (IPTG) wird bei 17°C über Nacht die Glykosyltransferase exprimiert. Darauf werden die Zellen bei 17 °C und 7.500 g für 20 min abzentrifugiert und in 0,1 M Phosphatpuffer mit 0,15 M Natriumchlorid resuspendiert. Dieser Kultur werden 1 (w/v)% Glucose sowie 0,2 mM Noreugenin zur Umsetzung hinzugefügt und bei 28°C weiter inkubiert. Nach 24 Stunden wird die Reaktion durch Abzentrifugation der Zellen bei 4°C und 10.000 g für 20 min beendet. Die Glykoside befinden sich nach Umsetzung im Überstand und werden mittels chromatographischer Aufreinigung isoliert. Der erste Trennschritt erfolgt auf einer RT 250 - 10 Pharm Prep Säule 100 RP-18e (10 µm) der Firma Merck. Über das Pumpensystem bei einem Flow von 10 mL/min auf der präpartiven HPLC Anlage 1260 Infinity von der Firma Agilent wird der gesamte Kulturüberstand auf der Säule angereichert und anschließend mit einem linearen Gradienten von steigender Acetonitrilkonzentration wieder von der Säule eluiert und dabei fraktioniert.

Der präparativen RP18-HPLC-Methode wie auch den analytischen RP18-Methoden liegt ein Stufengradient mit den Laufmitteln A (H₂O + 0,01% (v:v) Trifluouressigsäure) und dem Laufmittel B (Acetonitril + 0,01% (v:v) Trifluouressigsäure) zugrunde, der folgende Stufen aufweist:
0-10 min 5% B,
10-20 min 10% B,
20-30 min 15 % B,
30-40 min 20% B,
40-50 min 30% B,
50-60 min 100% B,
60-70 min 5% B.

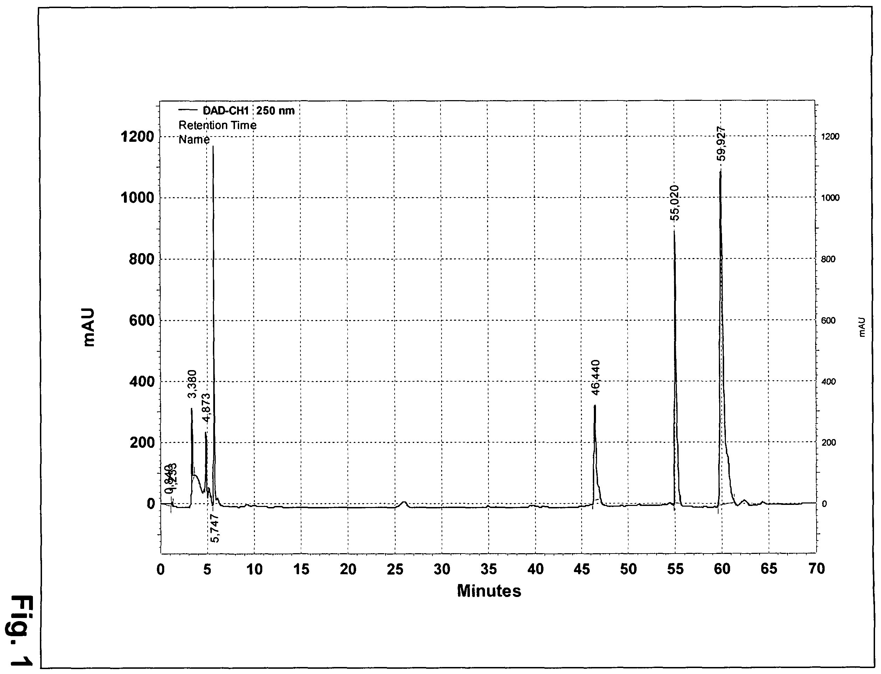

Bereits im ersten Chromatographieschritt können die einzelnen Verbingungen gut voneinander getrennt werden (siehe Fig. 1). Acetonitril wird mit einem Evaporator der Firma Büchi Labortechnik GmbH entfernt und die wässrige Lösung der einzelnen Noreugeninglykoside lyophilisiert. Das getrocknete Pulver wird in 30% Acetonitril (v:v) aufgenommen. Der zweite Aufreinigungsschritt zur Abtrennung verbleibender Verunreinigungen wird mit einer Hypersil Gold PFP (Pentafluorphenyl) Phase 250 x 4,6 mm der Firma Thermo Scientifc auf der VWR Hitachi LaChrom Elite Anlage durchgeführt. Ein isokratischer Lauf mit 30 % Acetonitril und einem Flow von 1 mL/min stellt die Zielsubstanzen in einer Reinheit von mehr als 95% dar. Die reinen Substanzen werden getrocknet und somit als lyophilisiertes Pulver erhalten.

Für die Analytik und die Prozesskontrolle werden analytische HPLC mit Diode Array Detektor (VWR Hitachi s.o.) und Massenspektrometrie angefertigt. Massenspektrometriedaten werden mit dem microOTOF-Q mit Elektronsprayionisierung (ESI) von Bruker Daltonik aufgenommen. Die Proben werden im *negative ion mode* gemessen und mit einer Spritzenpumpe bei einer Flussrate von 200 µL/min injiziert.

Fig. 1 zeigt den Überstand eines Fermentationsprozesses nach 6 Stunden Reaktionszeit. Der nichtglykosylierte Ausgangsstoff Noreugenin eluiert bei einer Retentionszeit von 60 min. Das Noreugenin 7-Rhamnosid hat eine Retentionszeit von 55 min und das hydrophilste Molekül ist das Noreugenin 5-Rhamnosid mit einer Retentionszeit von 46 min.

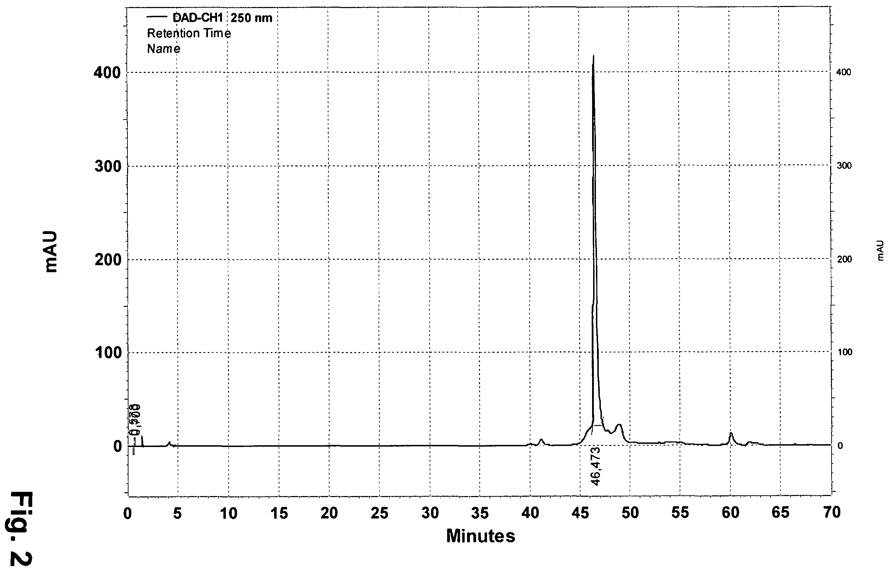

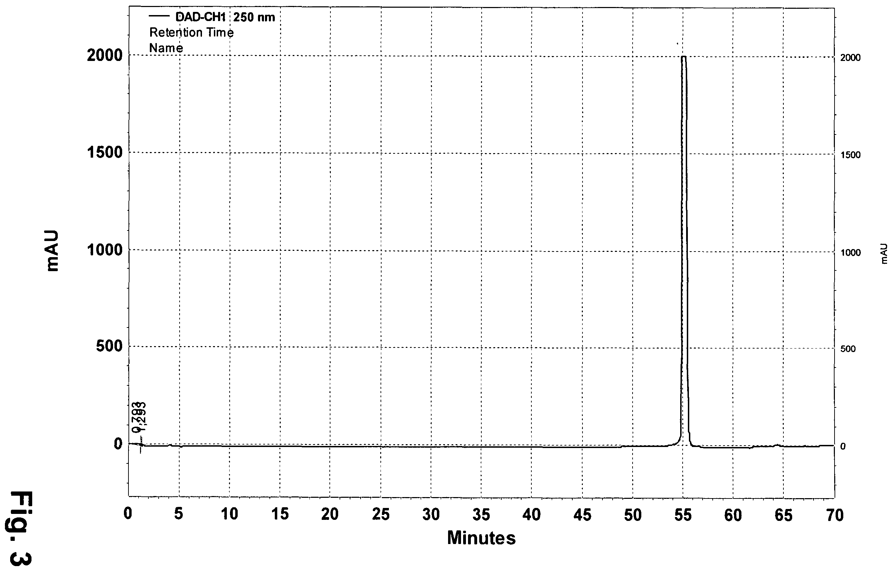

Die aufgereinigten Produkte sind in Fig. 2 und 3 abgebildet und zeigen einen Reinheitsgrad von > 95% bestimmt mit HPLC.

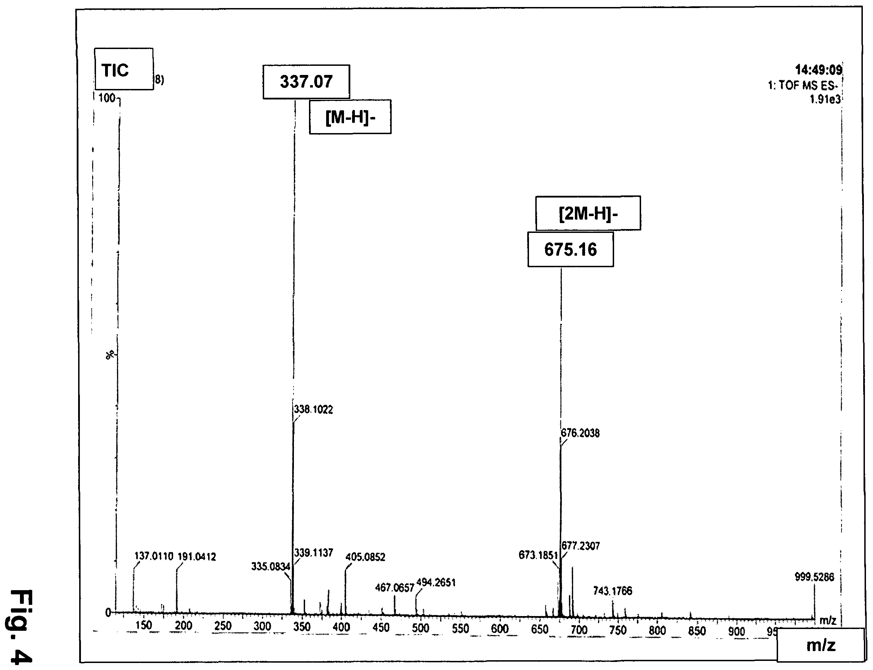

Zur eindeutigen Identifizierung wird weiterhin die Masse der vorgenannten Fraktionen bestimmt. Im *negative Ion Mode* stellen sich die Rhamnoside wie in Fig. 4 gezeigt dar. Das einfach geladene Molekül zeigt eine Molekülmasse von m/z 337. Zwei zusammen gelagerte Moleküle, die sich eine Ladung teilen, erscheinen im Massenspektrum als eine Masse von m/z 675.

NMR-Daten von Noreugenin 5-Rhamnosid (IIa):
1H-NMR (DMSO-δ, 400MHz): δ = 6,62 (d, 1H, J=2,2Hz), 6.58 (d, 1H, *J*=2,2Hz), 5.99 (d, 1H), 5.38 (d, 1H), 4,00 (s, 1H), 3.89-3.92 (dd, 1H, *J₁*=3,10Hz, *J₂*=9,2Hz), 3.49-3.54 (m, 7H), 3.36 (m, 1H), 2,30 (s, 3H).

## Patentansprüche

1. Verwendung mindestens einer Verbindung der Formel I oder II 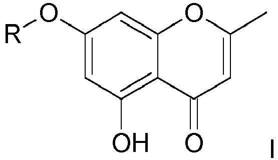 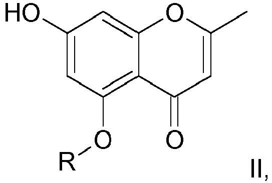 wobei die Reste R für Rhamnosyl stehen, wobei die Reste jeweils als D- oder L-Enantiomer und als α- oder β-Anomer vorliegen können,
als Selbstbräunungssubstanz.

2. Verwendung nach Anspruch 1 zur Steigerung der Melaninsynthese, Verbesserung des Melanintransports und/oder Verbesserung der Verteilung von Melanin in suprabasalen Schichten.

3. Verwendung nach Anspruch 1 oder 2, **dadurch gekennzeichnet, dass** die Verbindung der Formel I 7-O-α-L-rhamnosyl-noreugenin (7- [(6-deoxy-α-L-mannopyranosyl)oxy]-5-hydroxy-2-methyl-4*H*-1-Benzopyran-4-on) (Ib) ist und die Verbindung der Formel II 5-O-α-L-rhamnosylnoreugenin (5-[(6-deoxy-α-L-mannopyranosyl)oxy]-7-hydroxy-2-methyl-4*H*-1-Benzopyran-4-on) (IIa) ist.

4. Zubereitung enthaltend mindestens eine Verbindung der Formel I oder II 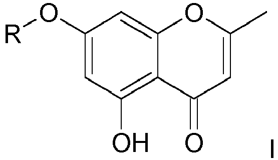 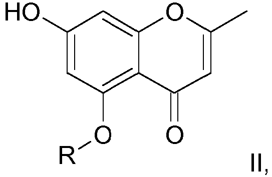 wobei R für Rhamnosyl steht, wobei die Reste jeweils als D- oder L-Enantiomer und als α- oder β-Anomer vorliegen können,
sowie einen kosmetisch geeigneten Träger.

5. Zubereitung nach Anspruch 4, **dadurch gekennzeichnet, dass** die Verbindung der Formel I 7-O-α-L-rhamnosyl-noreugenin (7- [(6-deoxy-α-L-mannopyranosyl)oxy]-5-hydroxy-2-methyl-4*H*-1-Benzopyran-4-on) (Ib) ist und die Verbindung der Formel II 5-O-α-L-rhamnosyl-noreugenin (5-[(6-deoxy-α-L-mannopyranosyl)oxy]-7-hydroxy-2-methyl-4*H*-1-Benzopyran-4-on) (IIa) ist.

6. Zubereitung nach Anspruch 4 oder 5, **dadurch gekennzeichnet, dass** die mindestens eine Verbindung der Formel I oder II in einer Menge von 0,01 bis 10 Gew.-% enthalten ist.

7. Zubereitung nach einem der Ansprüche 4 bis 6, **dadurch gekennzeichnet, dass** mindestens eine weitere Selbstbräunungssubstanz enthalten ist.

8. Verfahren zur Herstellung einer Zubereitung nach einem oder mehreren der Ansprüche 4 bis 7, **dadurch gekennzeichnet, dass** die mindestens eine Verbindung der Formel I oder II mit einem für topische Anwendungen geeigneten Träger vermischt wird.

9. Verbindung der Formel I oder II 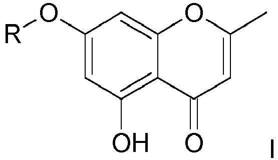 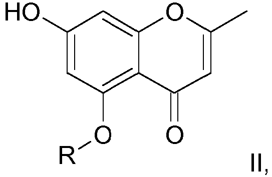 wobei die Reste R für Rhamnosyl stehen, wobei die Reste jeweils als D- oder L-Enantiomer und als α- oder β-Anomer vorliegen können.

10. Verbindung nach Anspruch 9, **dadurch gekennzeichnet, dass** die Verbindung der Formel I 7-O-α-L-rhamnosyl-noreugenin (7- [(6-deoxy-α-L-mannopyranosyl)oxy]-5-hydroxy-2-methyl-4*H*-1-Benzopyran-4-on) (Ib) ist und die Verbindung der Formel II 5-O-α-L-rhamnosyl -noreugenin (5-[(6-deoxy-α-L-mannopyranosyl)oxy]-7-hydroxy-2-methyl-4*H*-1-Benzopyran-4-on) (IIa) ist.

## Claims

1. Use of at least one compound of formula I or II 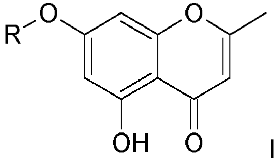 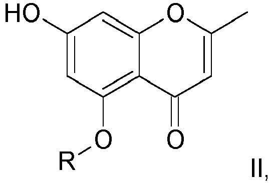 wherein the residues R stand for rhamnosyl, and wherein the residues can in each case be present as D or L enantiomer and as α or β anomer,
as a self-tanning substance.

2. Use according to claim 1 for increasing melanin synthesis, improving melanin transport and/or improving the distribution of melanin in suprabasal layers.

3. Use according to claim 1 or 2, **characterized in that** the compound of formula I is 7-O-α-L-rhamnosyl-noreugenin (7-[(6-deoxy-α-L-mannopyranosyl)oxy]-5-hydroxy-2-methyl-4*H*-1-Benzopyran-4-on) (Ib) and the compound of formula II is 5-O-α-L-rhamnosyl-noreugenin (5-[(6-deoxy-α-L-mannopyranosyl)oxy]-7-hydroxy-2-methyl-4*H*-1-Benzopyran-4-on) (Ila).

4. Preparation containing at least one compound of formula I or II 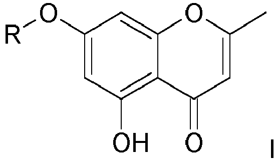 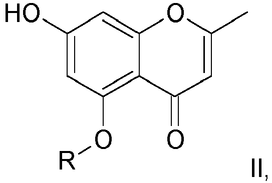 wherein R stands for rhamnosyl, and wherein the residues can in each case be present as D or L enantiomer and as α or β anomer,
as well as a cosmetically acceptable carrier.

5. Preparation according to claim 4, **characterized in that** the compound of formula I is 7-O-α-L-rhamnosyl-noreugenin (7-[(6-deoxy-α-L-mannopyranosyl)oxy]-5-hydroxy-2-methyl-4*H*-1-Benzopyran-4-on) (Ib) and the compound of formula II is 5-O-α-L-rhamnosyl-noreugenin (5-[(6-deoxy-α-L-mannopyranosyl)oxy]-7-hydroxy-2-methyl-4*H*-1-Benzopyran-4-on) (Ila).

6. Preparation according to claim 4 or 5, **characterized in that** it contains the at least one compound of formulae I or II in an amount of 0.01 to 10 % by weight.

7. Preparation according to one of claims 4 to 6, **characterized in that** it contains at least one further self-tanning substance.

8. Method for the production of a preparation according to one or more of claims 4 to 7, **characterized in that** the at least one compound of formula I or II is mixed with a carrier suitable for topical applications.

9. Compound of formula I or II 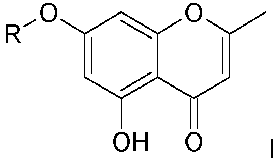 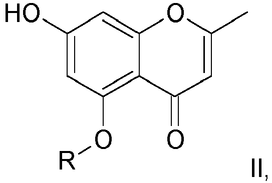 wherein the residues R stand for rhamnosyl, and wherein the residues can in each case be present as D or L enantiomer and as α or β anomer.

10. Compound according to claim 9, **characterized in that** the compound of formula I is 7-O-α-L-rhamnosyl-noreugenin (7-[(6-deoxy-α-L-mannopyranosyl)oxy]-5-hydroxy-2-methyl-4*H*-1-Benzopyran-4-on) (Ib) and the compound of formula II is 5-O-α-L-rhamnosyl-noreugenin (5-[(6-deoxy-α-L-mannopyranosyl)oxy]-7-hydroxy-2-methyl-4*H*-1-Benzopyran-4-on) (Ila).

## Revendications

1. Utilisation d'au moins un composé de formule I ou II 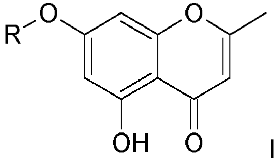 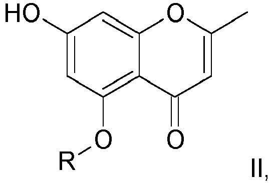 dans laquelle les radicaux R représentent le rhamnosyle, les radicaux pouvant respectivement être présents sous forme d'énantiomère D ou L et sous forme d'anomère α ou β en tant que substance autobronzante.

2. Utilisation selon la revendication 1 pour augmenter la synthèse de la mélamine, améliorer le transport de la mélamine et/ou améliorer la répartition de la mélanine dans les couches suprabasales.

3. Utilisation selon la revendication 1 ou 2, **caractérisée en ce que** le composé de formule I est de la 7-O-α-L-rhamnosyle-noreugénine (7- [(6-désoxy-α-L-mannopyranosyl)oxy]-5-hydroxy-2-méthyle-4*H*-1-benzopyrane-4-one) (Ib) et le composé de formule II est de la 5-O-α-L-rhamnosyle -noreugénine (5-[(6-désoxy-α-L-mannopyranosyl)oxy]-7-hydroxy-2-méthyle-4*H*-1-benzopyrane-4-one) (Ila).

4. Préparation contenant au moins un composé de formule I ou II 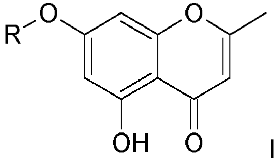 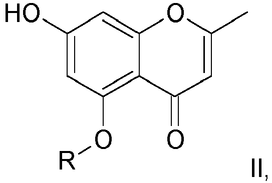 dans laquelle les radicaux R représentent le rhamnosyle, les radicaux pouvant respectivement être présents sous forme d'énantiomère D ou L et sous forme d'anomère α ou β, et un support adapté du point de vue cosmétique.

5. Préparation selon la revendication 4, **caractérisée en ce que** le composé de formule I est de la 7-O-α-L-rhamnosyle-noreugénine (7- [(6-désoxy-α-L-mannopyranosyl)oxy]-5-hydroxy-2-méthyle-4*H*-1-benzopyrane-4-one) (Ib) et le composé de formule II est de la 5-O-α-L-rhamnosyle-noreugénine (5-[(6-désoxy-α-L-mannopyranosyl)oxy]-7-hydroxy-2-méthyle-4*H*-1-benzopyrane-4-one) (Ila).

6. Préparation selon la revendication 4 ou 5, **caractérisée en ce que** l'au moins un composé de formule I ou II est contenu en une quantité de 0,01 à 10 % en poids.

7. Préparation selon une des revendications 4 bis 6, **caractérisée en ce qu'**au moins une autre substance autobronzante est contenue.

8. Procédé de réalisation d'une préparation selon une ou plusieurs des revendications 4 à 7, **caractérisé en ce que** l'au moins un composé de formule I ou II est mélangé avec un support adapté pour des applications topiques.

9. Composé de formule I ou II 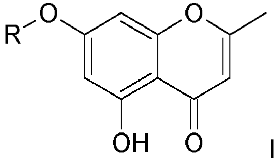 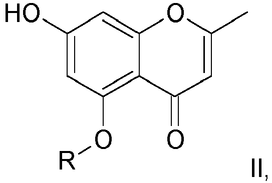 dans laquelle les radicaux R représentent le rhamnosyle, les radicaux pouvant respectivement être présents sous forme d'énantiomère D ou L et sous forme d'anomère α ou β.

10. Composé selon la revendication 9, **caractérisé en ce que** le composé de formule I est de la 7-O-α-L-rhamnosyle-noreugénine (7- [(6-désoxy-α-L-mannopyranosyl)oxy]-5-hydroxy-2-méthyle-4*H*-1-benzopyrane-4-one) (Ib) et le composé de formule II est de la 5-O-α-L-rhamnosyle-noreugénine (5-[(6-désoxy-α-L-mannopyranosyl)oxy]-7-hydroxy-2-méthyle-4*H*-1-benzopyrane-4-one) (Ila).
